# EUROPÄISCHE PATENTSCHRIFT

(11) **EP 4 198 117 B1**
(45) Veröffentlichungstag und Bekanntmachung des Hinweises auf die Patenterteilung: **08.10.2025**
(21) Anmeldenummer: 22212823.3
(22) Anmeldetag: 12.12.2022
(51) Int. Cl.: C12M 1/107, C12M 1/00

(54) **VORRICHTUNG UND VERFAHREN ZUR BIOLOGISCHEN METHANISIERUNG MIT IN EINEM KREISLAUF VERBUNDENER BEGASUNGS- UND ENTGASUNGSKOLONNE**
DEVICE AND METHOD FOR BIOLOGICAL METHANATION WITH A GAS- AND GAS-VENTING COLUMN CONNECTED IN A CIRCUIT
DISPOSITIF ET PROCÉDÉ DE MÉTHANISATION BIOLOGIQUE AVEC UNE COLONNE DE GAZÉIFICATION ET DE DÉGAZAGE RELIÉE EN CIRCUIT

(30) Priorität: 14.12.2021 DE 102021133102
(43) Veröffentlichungstag der Anmeldung: 21.06.2023
(73) Patentinhaber: Hochschule Offenburg, 77652 Offenburg (DE)
(72) Erfinder: Hochberg, Ulrich, 76199 Karlsruhe (DE); Zell, Christiane, 76275 Ettlingen (DE); Schaub, Martin, 77933 Lahr (DE); Bieri, Markus, CH-8803 Rüschlikon (CH); Stalder, Markus, CH-4537 Wiedlisbach (CH); Haitz, Fabian, 77746 Schutterwald (DE); Scharffenberg, Manuel, 77960, Seelbach (DE)
(74) Vertreter: Eder Schieschke & Partner mbB

(56) Entgegenhaltungen:
- EP-A2- 0 154 334
- EP-B1- 3 013 937
- WO-A1-2020/208042
- DE-A1- 102018 117 281
- DE-A1- 102020 109 419

## Beschreibung

Die vorliegende Erfindung betrifft eine Vorrichtung zur biologischen Methanisierung von Kohlenstoffdioxid mittels methanogener Mikroorganismen mit den Merkmalen des Patentanspruchs 1. Weiterhin betrifft die vorliegende Erfindung ein Verfahren zur biologischen Methanisierung von Kohlenstoffdioxid in einer Vorrichtung mittels methanogener Mikroorganismen mit den Merkmalen des Patentanspruchs 9. Anstelle von Kohlenstoffdioxid kann erfindungsgemäß auch Kohlenstoffmonoxid eingesetzt werden, beispielsweise aus der Pyrolyse von Pflanzenresten oder der Hochtemperatur-Co-Elektrolyse.

Alternativ zur Speicherung von elektrischer Energie in Akkumulatoren wird aus Gesichtspunkten des einfacheren Transports und längerfristiger Speicherung großer Mengen von Energie das Konzept "power-to-gas" immer wichtiger. Nach diesem Konzept wird beispielsweise überschüssiger elektrischer Strom aus beispielsweise regenerativen Quellen zur elektrolytischen Spaltung von Wasser in Wasserstoff verwendet, der dann chemisch oder biologisch durch Reaktion mit Kohlenstoffmonoxid und/oder Kohlenstoffdioxid in speicherbares Methan umgewandelt werden kann. Die chemische Reaktion wird bei Temperaturen von über 180 °C und hohen Drücken erzwungen und nur durch relativ anspruchsvolle Katalysatoren ermöglicht. Dagegen kann die biologische Umwandlung von Wasserstoff und Kohlenstoffdioxid in Methan durch spezielle Mikroorganismen schon bei Normaldruck und Temperaturen im Bereich von 25-70 °C erfolgen. In natürlichen, anaeroben, wässrigen Ökosystemen wie Sümpfen, Gewässersedimenten oder überfluteten Böden wird von Konsortien mehrerer Mikroorganismen über eine Abbaukette Methan aus organischem Material wie Pflanzen- und Tierresten gebildet. In einer ersten Reaktion findet hier eine Hydrolyse von Polymeren zu Oligomeren oder Monomeren statt. In einem zweiten Schritt, der Acidogenese, entstehen neben Carbonsäuren, Ethanol, Schwefelwasserstoff und Ammoniak auch Essigsäure, Wasserstoff und Kohlenstoffdioxid. Im dritten Schritt, der Acetogenese, werden niedere Fett- und Carbonsäuren sowie Alkohole zu Essigsäure umgesetzt. Im vierten Schritt, der Methanogenese, wird Essigsäure und/oder Kohlenstoffdioxid und Wasserstoff durch methanogene Mikroorganismen, den Archaeen, zu Methan umgesetzt.

Dieser 4. Schritt kann gezielt zur Speicherung von Energie in Form von Methan genutzt werden, wenn dem entsprechenden Medium, das methanogene Mikroorganismen enthält, Wasserstoff beigesetzt wird. Damit wird das überschüssige Kohlenstoffdioxid zu Methan umgesetzt. Alternativ kann der Methanisierungsschritt auch separat durchgeführt werden. In einem wässrigen Medium, das lediglich geringe Konzentrationen an essentiellen Mikro- und Makroelementen enthält, wird nämlich auch extern zugeführtes Kohlenstoffdioxid oder Kohlenstoffmonoxid mit H₂ durch die methanogenen Archaeen zu Methan umgesetzt.

Solche Vorrichtungen, bei denen der genannte 4. Schritt zur Energiespeicherung in Form von Methan genutzt wird, sind bereits bekannt. Problematisch hat sich hier immer die Löslichkeit von Wasserstoff im wässrigen Medium herausgestellt, das die methanogenen Mikroorganismen enthält. Das "in Lösung bringen" des Wasserstoffs ist hierbei sehr energieaufwändig.

Weiterhin ist der pH-Wert des wässrigen Mediums abhängig von der Menge an gelöstem Kohlenstoffdioxid im System. Eine Erhöhung des Kohlenstoffdioxidgehalts erniedrigt den pH-Wert des wässrigen Mediums, während ein Absenken des Kohlenstoffdioxidgehalts den pH-Wert erhöht. Beides kann den für die Mikroorganismen geeigneten pH-Wertbereich ungünstig beeinflussen.

Die Anwesenheit von Kohlenstoffmonoxid/Kohlenstoffdioxid im System ist aber insbesondere dann wichtig, wenn im Biogasprozess auch Wasserstoff zugeführt wird, da dieser dann sofort von den Mikroorganismen verstoffwechselt werden kann. Ist der Kohlenstoffmonoxid-/Kohlenstoffdioxidgehalt im wässrigen Medium zu niedrig, kommt es zur Wasserstoffakkumulation, die den anaeroben Abbau hemmt. Es ist deshalb wichtig, die Kohlenstoffmonoxid-/Kohlenstoffdioxidkonzentration im System auf den idealen Bereich einzustellen.

Eine große Herausforderung bei der biologischen Methanisierung ist - wie beschrieben - die geringe Löslichkeit von Wasserstoff in wässrigen Medien. Beim Einsatz konventioneller Begasungstechniken kann der eingebrachte Wasserstoff aufgrund des unzureichenden Gas-/Flüssig-Transfers nicht vollständig von der Mikrobiologie umgesetzt werden und geht weitgehend ungenutzt ins Biogas über. Dies ist nicht nur energetisch ungünstig, sondern kann auch bei einer Einspeisung ins Erdgasnetz problematisch sein, da ein Grenzwert von wenigen Vol.-% Wasserstoff nicht überschritten werden sollte.

Um eine homogene Verteilung von Wasserstoff und Kohlenstoffmonooxid/Kohlenstoffdioxid in solchen wässrigen Medien zu ermöglichen, wird im Stand der Technik zumeist eine mechanische Umwälzung des Substrats während der Begasung vorgeschlagen. Durch die mechanische Umwälzung wird jedoch auch eine erhebliche Menge an Energie verbraucht. Weiterhin sind Reaktoren mit Umwälzungsvorrichtungen aufgrund ihres komplizierteren Aufbaus schwieriger zu reinigen. Alternativ dazu wurden im Stand der Technik auch Reaktoren vorgeschlagen, in denen Wasserstoff oder Synthesegas über eine Mehrzahl von fein verteilten Begasungseinheiten in den Reaktor gegeben wird.

Die EP 3 013 937 B1 betrifft in einem ersten Aspekt eine Vorrichtung zum Herstellen von Methan mittels methanogener Mikroorganismen durch Umsetzung von H₂ und CO₂, aufweisend: mindestens einen Reaktor; ein wässriges Medium, welches in dem mindestens einen Reaktor bereitgestellt ist, wobei sich die methanogenen Mikroorganismen in dem wässrigen Medium befinden; eine Zuführungseinrichtung, welche zum Einleiten von H₂ und CO₂ in den mindestens einen Reaktor eingerichtet ist, wobei das H₂ und CO₂ darin ein gasförmiges Gemisch bilden; eine Reaktionssteigerungsvorrichtung, welche eingerichtet ist zum Vergrößern der Kontaktoberfläche zwischen dem wässrigen Medium mit den methanogenen Mikroorganismen und dem gasförmigen Gemisch; und eine Rückführungseinrichtung, welche eingerichtet ist zum Rückführen mindestens eines Teils des sich im Reaktor ansammelnden Gases, wobei die Rückführungsrate des sich im Reaktor ansammelnden Gases größer ist als die Zuführungsrate des Substratgases in den Reaktor. In einem zweiten Aspekt betrifft die EP 3 013 937 B1 die Verwendung einer erfindungsgemäßen Vorrichtung zur Herstellung von Methan mittels methanogener Mikroorganismen durch Umsetzung von H₂ und CO₂. In einem dritten Aspekt betrifft die EP 3 013 937 B1 ein Verfahren zur Herstellung von Methan in einer erfindungsgemäßen Reaktorvorrichtung mittels methanogener Mikroorganismen, die sich in der Reaktorvorrichtung in einem wässrigen Medium befinden, durch Umsetzung von H₂ und CO₂ als Reaktionsgasgemisch, wobei bei der Umsetzung die Kontaktoberfläche zwischen dem wässrigen Medium mit den methanogenen Mikroorganismen und dem gasförmigen Gemisch mittels einer Reaktionssteigerungsvorrichtung vergrößert wird und wobei mittels einer Rückführungseinrichtung mindestens ein Teils des sich im Reaktor ansammelnden Gases in die Reaktorvorrichtung zurückgeführt wird, wobei die Rückführungsrate des sich im Reaktor ansammelnden Gases größer ist als die Zuführungsrate des Reaktionsgemisches in den Reaktor.

Die DE 102018 117281 A1 betrifft eine Vorrichtung zur biologischen Methanisierung von Kohlenstoffdioxid mittels methanogener Mikroorganismen durch Umsetzung von Wasserstoff und Kohlenstoffdioxid die Folgendes aufweist: (a) einen Reaktor; (b) ein in dem Reaktor bereitgestelltes Medium mit methanogenen Mikroorganismen; (c) eine Zuführeinrichtung zum Zuführen eines H-enthaltenden Gases in das Medium; wobei die Zuführeinrichtung eine Mehrzahl an Gasführungseinheiten umfasst, wobei jede der Gasführungseinheiten eine Mehrzahl an Begasungseinheiten aufweist, die jeweils eine Vielzahl an Auslassöffnungen zum Zuführen des H-enthaltenden Gases in das Medium aufweist, wobei die Zuführeinrichtung derart ausgestaltet ist, dass das Zuführen des H-enthaltenden Gases durch sukzessive Beaufschlagung der Gasführungseinheiten erfolgen kann. Weiterhin betrifft die Erfindung auch ein Verfahren zur biologischen Methanisierung von Kohlenstoffdioxid in einer Reaktorvorrichtung mittels methanogener Mikroorganismen als Teil eines in einem Reaktor bereitgestellten Mediums, dadurch gekennzeichnet, dass ein H-enthaltendes Gas über eine Mehrzahl an Gasführungseinheiten dem Medium zugeführt wird, wobei jede der Gasführungseinheiten eine Mehrzahl an Begasungseinheiten aufweist, die jeweils eine Vielzahl an Auslassöffnungen aufweisen, wobei das Zuführen des H-enthaltenden Gases durch sukzessive Beaufschlagung der Gasführungseinheiten erfolgt. Dem H-enthaltenden Medium kann nach Bedarf Kohlenstoffdioxid beigemischt werden.

Die EP 0 154 334 A2 betrifft ein Verfahren zur Durchführung von Reaktionen und Stoffaustauschprozessen in heterogenen fluiden Systemen. Bei diesen stellt sich häufig das Problem, eine Reaktionskomponente oder auch einen Hilfsstoff nach der Reaktions- bzw. Austauschstufe von der Reaktionslösung abzutrennen und wieder in den Prozess zurückzuführen. Dies ist insbes. bei Anwendung von Druck und hohen Temperaturen mit einem großen Aufwand in Form zusätzlicher aufwendiger Vordereinrichtungen verbunden. Zur Behebung dieses Nachteils sieht die Erfindung vor, einen Reaktor mit Rührkesselcharakteristik als erster Stufe mit einem Reaktor mit Rohrcharakteristik als zweiter Stufe nach Art eines kommunizierenden hydraulischen Systems im Bereich ihrer oberen und unteren Enden durch Rohrleitungen miteinander zu verbinden.

Es war deshalb die Aufgabe der vorliegenden Erfindung, eine Vorrichtung und ein Verfahren zur biologischen Methanisierung von Kohlenstoffmonoxid/Kohlenstoffdioxid bereitzustellen, bei denen Methan in hohen Konzentrationen vorliegt. Zudem soll während der Umsetzung von Wasserstoff und Kohlenstoffmonoxid/Kohlenstoffdioxid zu Methan im wässrigen Medium weniger Energie als mit herkömmlichen Anlagen benötigt und eine Vereinfachung hinsichtlich bekannter Vorrichtungen und Verfahren erreicht werden. Dennoch muss die Zufuhr des Wasserstoffs derart erfolgen, dass er ausreichend im Medium gelöst wird. Weiterhin soll der Kohlenstoffdioxidgehalt so einstellbar sein, dass er im für die Mikroorganismen optimalen Bereich liegt, und dass es zu keiner Wasserstoffakkumulation kommt. Auch ist es für eine kontinuierliche und hohe Methanbildungsrate weiterhin entscheidend, dass zu jedem Zeitpunkt hochleistungsfähige Mikroorganismen in einer optimalen Konzentration im System vorhanden sind. Unterschiedliche chemische und biologische Faktoren führen jedoch während des Betriebs in unterschiedlichem Ausmaß zu Abweichungen von dieser Idealsituation. Durch das gebildete Reaktionswasser kommt es zudem zu einer Verdünnung der Mikroorganismen. Unter anderem können auch zu hohe oder zu niedrige pH-Werte oder Temperaturen, die fehlende Zufuhr noch nicht genau bekannter essentieller Spurenelemente oder die Zufuhr inhibierender Stoffe bei der Verwendung komplexer Substrate die Biologie schädigen. Erfolgt die Methanisierung direkt im Biogasprozess kann es zudem bei zu hohen Beladungsraten oder einer zu hohen Wasserstoff-Zufuhr auch zu einer Schädigung vor allem der acetogenen Mikroorganismen kommen. Werden genetisch optimierte Hochleistungsstämme verwendet, kommt es im Laufe der Zeit zu Mutationen und/oder Kontaminationen. Dann müssen neue Chargen der Hochleistungsstämme eingebracht werden. Zum Erreichen hoher Produktivitätsraten ist daher eine kontrollierte Einstellung der Mikroorganismenkonzentration erforderlich.

Zur Lösung der gestellten Aufgabe(n) stellt die vorliegende Erfindung eine Vorrichtung mit den Merkmalen des Patentanspruchs 1 bereit. Die erfindungsgemäße Vorrichtung eignet sich zur biologischen Methanisierung von Kohlenstoffdioxid mittels methanogener Mikroorganismen durch Umsetzung von Wasserstoff und Kohlenstoffmonoxid und/oder Kohlenstoffdioxid. Die erfindungsgemäße Vorrichtung weist Folgendes auf:
(a) eine Begasungskolonne und eine Entgasungskolonne, jeweils mit einer Bodenseite und einer der Bodenseite gegenüberliegenden oberen Seite;
(b) ein in der Begasungskolonne und der Entgasungskolonne bereitgestelltes Medium mit methanogenen Mikroorganismen;
(c) eine Zuführeinrichtung zum Zuführen eines Wasserstoff enthaltenden Gases in das Medium der Begasungskolonne, wobei die Zuführeinrichtung im Bereich der Bodenseite der Begasungskolonne angeordnet ist;
(d) eine Abführeinrichtung zum Abführen eines Methan enthaltenden Gases aus der Entgasungskolonne;
(e) eine Verbindungsleitung zwischen Begasungskolonne und Entgasungskolonne im Bereich der Bodenseiten;
(f) eine Pumpe zum Überführen von Medium über die Verbindungsleitung von der Begasungskolonne in die Entgasungskolonne; und
(g) eine Rückführleitung zwischen der Begasungskolonne und der Entgasungskolonne im Bereich der oberen Seiten zum Rückführen von Medium aus der Entgasungskolonne in die Begasungskolonne.

Die Begasungskolonne und Entgasungskolonne sind durch die Verbindungsleitung im Bereich der Bodenseiten und durch die Rückführleitung im Bereich der oberen Seiten miteinander verbunden. Das in beiden Kolonnen enthaltene Medium kann somit in einem Kreislauf zwischen der Begasungskolonne und der Entgasungskolonne bewegt werden. Die Pumpe ist so ausgebildet, dass sie das Medium über die Verbindungsleitung von der Begasungskolonne in die Entgasungskolonne überführen kann. Über die Rückführleitung kann das von der Begasungskolonne in die Entgasungskolonne gepumpte Medium wieder von der Entgasungskolonne in die Begasungskolonne zurückgeführt werden. Da der Druck in dem Medium in der Begasungskolonne im Bereich der Bodenseite höher ist als im Bereich der oberen Seite, kann das Medium in diesem Bereich mehr Wasserstoff enthaltendes Gas aufnehmen. Aus dem gleichen Grund kann im Bereich der Bodenseite der Begasungskolonne auch mehr gebildetes Methan im Medium gelöst vorliegen, als dies im Bereich der oberen Seite der Begasungskolonne der Fall ist. Da die Verbindungsleitung im Bereich der Bodenseiten der beiden Kolonnen angeordnet ist, wird durch den hier höheren Druck Medium mit einem höheren Gehalt an gelöstem Methan von der Begasungskolonne in die Entgasungskolonne überführt. Da der Druck in der Entgasungskolonne durch das darin aufsteigende Medium abnimmt, nimmt auch die Löslichkeit des Methans im Medium hierbei ab. Auf diese Weise kann über die Abführeinrichtung in der Entgasungskolonne das aus dem Medium ausgasende Methan abgeführt werden. Nicht bereits in der Begasungskolonne verwerteter Wasserstoff wird in der Entgasungskolonne umgesetzt. Daher ist relativ wenig Wasserstoff in dem enthaltenen Produktgas vorhanden. In anderen Worten wird hier die Druckdifferenz in den beiden Kolonnen ausgenutzt, um möglichst wenig Wasserstoff und möglichst viel Methan im Produktgas zu erhalten.

Erfindungsgemäß ist die Zuführeinrichtung im Bereich der Bodenseite der Begasungskolonne angeordnet. Auf diese Weise strömt das in die Begasungskolonne eingeführte Wasserstoff enthaltende Gas in der Begasungskolonne aufwärts entgegen dem darin abwärts strömenden Medium. Die methanogenen Mikroorganismen im Medium setzen darin enthaltenes Kohlenstoffmonoxid und/oder Kohlenstoffdioxid unter Verbrauch des zugeführten Wasserstoffs in Methan um. Da das Methan besser als Wasserstoff im Medium löslich ist und zudem durch den höheren Druck im Bereich der Bodenseite der Begasungskolonne das Methan dort besser löslich als im Bereich der oberen Seite der Begasungskolonne ist, wird überwiegend das gelöste Methan mit dem in die Entgasungskolonne überführten Medium überführt.

Weiterhin hat die vorzugsweise parallele Anordnung der Begasungskolonne und Entgasungskolonne den Vorteil, dass die Pumpe unter relativ geringem Energieaufwand eine Kreislaufbewegung des Mediums bewirken kann, bei der die Höhe der Flüssigkeitssäule in der Entgasungskolonne nur etwas höher als in der Begasungskolonne ist. Durch diesen Unterschied in der Höhe der Flüssigkeit (sogenannter Füllstandunterschied) der Entgasungskolonne und der Begasungskolonne kann das Medium im Bereich der oberen Seiten der Kolonnen durch die Rückführleitung von der Entgasungskolonne in die Begasungskolonne zurückgeführt werden. Die Pumpe zwischen Begasungskolonne und Entgasungskolonne muss dabei nur den Füllstandunterschied zwischen den beiden Kolonnen sowie geringfügige Strömungsdruckverluste und Druckdifferenzen im Gasbereich der Kolonnen überwinden. Der Energiebedarf der Pumpe kann somit relativ klein und der Energieaufwand zur biologischen Methanisierung auf diese Weise äußerst gering gehalten werden.

Es ist weiterhin erfindungsgemäß bevorzugt, dass am oberen Ende des Mediums in der Entgasungskolonne Atmosphärendruck herrscht.

Die Abführeinrichtung ist in der erfindungsgemäßen Vorrichtung vorzugsweise im Bereich der oberen Seite der Entgasungskolonne angeordnet. Der Grund hierfür liegt darin, dass das Methan hier aufgrund des geringeren hydraulischen Drucks im Bereich der oberen Seite der Entgasungskolonne eine geringere Löslichkeit im Medium aufweist, sodass es aus diesem ausgasen und durch die Abführeinrichtung abgeführt werden kann. Hierfür kann die Entgasungskolonne im Bereich der oberen Seite zusätzlich zur vorzugsweise als Rohrleitung ausgebildeten Abführeinrichtung ein Rohr mit Zackenwehr oder Zackenwehrkaskade aufweisen.

Das Medium ist vorzugsweise ein (komplexes) wässriges Medium und enthält neben Wasser und Substrat sowie gegebenenfalls genetisch optimierte methanogene Mikroorganismen, insbesondere hydrogenotrophe methanogene Mikroorganismen in Rein- oder Mischkultur. Das Substrat umfasst vorzugsweise Kohlenstoffmonoxid und/oder Kohlenstoffdioxid und Wasserstoff als Hauptkomponenten für die Energiegewinnung der methanogenen Mikroorganismen und kann daneben noch Spurenelemente und/oder weitere komplexe Zusätze, wie bspw. Jauche, enthalten. Methanogene Mikroorganismen im Sinne der vorliegenden Erfindung sind solche Mikroorganismen, die aus Kohlenstoffdioxid und Wasserstoff oder aus Essigsäure Methan herstellen können, d. h. Methanbildner oder methanbildende Mikroorganismen sind. Hydrogenotrophe methanogene Mikroorganismen im Sinne der vorliegenden Erfindungen sind solche methanogene Mikroorganismen, die aus Kohlenstoffdioxid und Wasserstoff Methan bilden. Methanogene Mikroorganismen gehören zur Domäne Archaea und darin zum Stamm Euryarchaeota. Methanogene Mikroorganismen gehören u. a. zu den Ordnungen Methanobacteriales, Methanococcales, Methanomicrobiales, Methanocellales, Methanosarcinales und Methanopyrales. Im Rahmen der vorliegenden Erfindung ist vorgesehen, dass methanogene Mikroorganismen aus allen Ordnungen stammen können, aber nicht müssen. Es ist daher bevorzugt, dass die methanogenen Mikroorganismen eine Misch- oder Reinkultur verschiedener Mikroorganismen des Stamms Euryarchaeota sind. Das Medium kann neben den genannten Mikroorganismen aber auch sämtliche Mikroorganismen enthalten, die in einem Reaktor einer Biogasanlage enthalten sind.

In einer weiteren Ausgestaltung weist die erfindungsgemäße Vorrichtung vorzugsweise eine Zuführeinrichtung zum Zuführen eines Kohlenstoffdioxid oder Kohlenstoffmonoxid enthaltenden Gases (z. B. aus der Pyrolyse von Pflanzenresten oder einer Co-Elektrolyse) oder einer organischen Vorläuferverbindung davon, die durch im Medium enthaltene Mikroorganismen in Kohlenstoffmonoxid und/oder Kohlenstoffdioxid überführt wird, in das Medium der Begasungskolonne auf, wobei die Zuführeinrichtung im Bereich der Bodenseite der Begasungskolonne angeordnet ist. Als Vorläuferverbindungen kommen erfindungsgemäß vorzugsweise die folgenden in Betracht, z. B. zucker-, stärke- oder cellulosehaltige industrielle Reststoffe und Abwässer wie z. B. Molke, Melasse, Abfälle der Hefe- und Sojaprodukteherstellung, der Mais- oder Kartoffelstärkeindustrie, der Papierindustrie etc, sowie sonstige biomassehaltige Abfälle. Als Mikroorganismen zur Überführung der Vorläuferverbindungen kann das Medium zusätzlich zu den zuvor genannten Mikroorganismen folgende aufweisen: Vertreter von Bacteria (v.a. Firmicutes, Bacteroidetes, Actinobacteria, Fibrobacteres/Acidobacteria sowie Crenarchaeota).

Weiterhin weist die erfindungsgemäße Vorrichtung vorzugsweise eine Einrichtung zum Abführen von Flüssigkeit mit Rückhalt von Mikroorganismen auf. Dies ermöglicht das Entfernen von bei der biologischen Methanisierung entstehendem Reaktionswasser. Dies hat den Vorteil, dass die Stoffmenge innerhalb der Vorrichtung in einem konstanten Rahmen gehalten werden kann. Ansonsten würde das bei der biologischen Methanisierung entstehende Reaktionswasser stetig die Stoffmenge innerhalb der Vorrichtung erhöhen. Um das Reaktionswasser aus dem Mikroorganismen enthaltenden Medium abzutrennen, kann die genannte Einrichtung ein Filtrationsmodul, beispielsweise ein Membranmodul, sein, das so ausgebildet ist, dass das Reaktionswasser, aber nicht Mikroorganismen, abgeführt werden. Erfindungsgemäß erfolgt die Abführung des Reaktionswassers, sobald der Füllstandunterschied zwischen Entgasungskolonne und Begasungskolonne einen vorgegebenen Maximalwert überschreitet. Zusammen mit einer Zuführleitung für Mikroorganismen und einer Abführleitung ohne Rückhaltung der Mikroorganismen ist die kontrollierte Einstellung einer optimalen Mikroorganismenkonzentration möglich.

In einer weiteren Ausgestaltung weist die erfindungsgemäße Vorrichtung vorzugsweise im Bereich der oberen Seite der Begasungskolonne eine Gasentnahmeleitung auf, mit der nicht umgesetzter Wasserstoff, nicht gelöstes Methan und Kohlenstoffmonoxid und/oder Kohlenstoffdioxid aus dem Bereich der oberen Seite der Begasungskolonne in den Bereich der Bodenseite der Begasungskolonne geführt werden. Die dabei abgeführte Gasmenge dient der Vereinfachung der Regelung: im Prinzip wird so viel Wasserstoff zugeführt, dass er nahezu vollständig umgesetzt werden kann. Die Regelung der erfindungsgemäßen Vorrichtung ist jedoch stabiler, wenn immer ein kleiner Teil des entstehenden Gases im Bereich der oberen Seite der Begasungskolonne abgezogen und im Bereich der Bodenseite der Begasungskolonne wieder aufgegeben wird.

Prinzipiell sind mehrere Betriebsarten der erfindungsgemäßen Vorrichtung möglich, von denen die zwei folgenden bevorzugt sind:
(a) Methanisierung einer Gasmischung von Methan und Kohlenstoffdioxid, und
(b) Methanisierung von reinem Kohlenstoffdioxid, Kohlenmonoxid oder einem Gemisch von beiden.

Die Betriebsart (a) bietet sich erfindungsgemäß an, wenn der Gasstrom der Edukte aus Verkehrungsanlagen von organischen Reststoffen, aus Faultürmen von Kläranlagen, Deponieabgasen etc. kommt. Hier ist die Regelung anspruchsvoller, da das zugegebene Kohlenstoffdioxid zunächst nicht unabhängig vom Methan geregelt werden kann.

Bei Betriebsart (b) kann der Mengenstrom an zu methanisierendem Kohlenstoffdioxid oder Kohlenstoffmonoxid in gewissem Rahmen frei gewählt werden. Kohlenstoffdioxid kann dann aus anderen Quellen kommen, beispielsweise aus Abluft aus der Bier- und Weinherstellung, aus der Abluft von Zementwerken oder aus Anlagen zur Kohlenstoffdioxidabtrennung aus der Atmosphäre. Gemische aus Kohlenstoffdioxid und Kohlenmonoxid können z. B. aus einer Pyrolyse stammen.

Weiterhin ist es erfindungsgemäß bevorzugt, dass die Pumpleistung der eingesetzten Pumpe (zum Transport des Mediums von der Begasungskolonne in die Entgasungskolonne) mindestens so groß ist, dass der dabei entstehende Volumenstrom multipliziert mit der Löslichkeitsdifferenz des Methans (Oberseite/Bodenseite der Begasungskolonne) in dem Medium größer ist als die Menge des produzierten Methans. Ist Kohlenstoffdioxid zu methanisieren, das in einem zugeführten Gemisch aus Kohlenstoffdioxid und Methan anfällt, so sollte die Pumpleistung aufgrund des schon im Eduktstrom vorhandenen Methans vergrößert werden.

Die Zudosierung von Kohlenstoffdioxid kann erfindungsgemäß auch durch den pH-Wert gesteuert werden: Bei der Methanisierung steigt prinzipiell der pH-Wert. Damit der pH-Wert nicht über einen vorgegebenen Wert von beispielsweise pH = 8,0 steigt, wird bei steigendem pH-Wert die Menge des zudosierten Kohlenstoffdioxids erhöht. Bei zu niedrigem pH-Wert, beispielsweise kleiner als pH = 7,5, sinkt die Methanisierungsrate. In diesem Fall muss die Kohlenstoffdioxidmenge verringert oder eine Kationen enthaltende Lösung zudosiert werden. Ergänzend kann der optimale pH-Bereich auch durch puffernde sowie sauer bzw. ggf. alkalisch wirkende Stoffe, bevorzugt Rest- und Abfallstoffe eingestellt werden. Beim Betrieb der Anlage als anaerobe Vergärungsanlage können dies auch die Substrate sein.

Die Höhe der Kolonnen ist vorzugsweise so gewählt, dass der Füllstand des Mediums in der Begasungskolonne mehr als 10 m, stärker bevorzugt mehr als 20 m und noch stärker mehr als 30 m ist. Der Vorteil einer größeren Höhe liegt darin, dass der hydrostatische Druck im Bereich der Bodenseite größer ist, und somit mehr Wasserstoff und Methan gelöst werden kann.

Die Begasungskolonne und/oder die Entgasungskolonne können zudem flüssigkeitsdurchlässige Füllkörper wie beispielsweise Raschingringe oder grobfaserige Fließe aufweisen. Dadurch kann der Stoffübergang verbessert, und Mikroorganismen können immobilisiert werden.

Neben der genannten Einrichtung zum Abführen von Flüssigkeit mit Rückhalt von Mikroorganismen kann die erfindungsgemäße Vorrichtung auch eine Einrichtung zum Abführen von Flüssigkeit ohne Rückhalt von Mikroorganismen aufweisen. Daneben kann die erfindungsgemäße Vorrichtung auch eine Zuführeinrichtung für eine Mikroorganismen enthaltende Flüssigkeit aufweisen, die vorzugsweise Mikroorganismen in hochkonzentrierter Form aufweist. Diese Zu- und Abführeinrichtungen ermöglichen eine kontrollierte Einstellung der Mikroorganismenkonzentration im Medium.

Die vorliegende Erfindung betrifft auch ein Verfahren zur biologischen Methanisierung von Kohlenstoffmonoxid und/oder Kohlenstoffdioxid in einer Vorrichtung mittels methanogener Mikroorganismen als Teil eines in der Vorrichtung bereitgestellten Mediums. Das Verfahren ist dadurch gekennzeichnet, dass das Medium in einem Kreislauf über eine Begasungskolonne und eine Entgasungskolonne geführt wird, wobei die Kolonnen über eine Verbindungsleitung im Bereich ihrer Bodenseiten und über eine Rückführleitung im Bereich der den Bodenseiten gegenüberliegenden oberen Seiten miteinander verbunden sind, worin das Medium sich in der Begasungskolonne absteigend und in der Entgasungskolonne aufsteigend bewegt, worin dem Medium im Bereich der Bodenseite der Begasungskolonne ein Wasserstoff enthaltendes Gas zugeführt wird.

Da die Löslichkeit proportional zum Druck ist, kann durch die Zuführung des Wasserstoff enthaltenden Gases im Bereich der Bodenseite der Begasungskolonne sich dort mehr Wasserstoff im Medium lösen.

Das sich bildende Methan wird zumindest teilweise in dem Medium gelöst und über die Verbindungsleitung in die Entgasungskolonne transportiert. Durch die aufsteigende Bewegung des Mediums in der Entgasungskolonne wird das Methan in Richtung der oberen Seite der Entgasungskolonne transportiert. Durch die Druckverminderung des Mediums in Richtung der oberen Seite der Entgasungskolonne kann das Methan aus dem Medium ausgasen und am oberen Ende der Entgasungskolonne abgeführt werden. Die absteigende Bewegung des Mediums in der Begasungskolonne und die aufsteigende Bewegung des Mediums in der Entgasungskolonne wird vorzugsweise durch eine Pumpe bewirkt. Dabei ergibt sich zwischen der Entgasungskolonne und der Begasungskolonne ein Füllstandunterschied des darin enthaltenen Mediums, d. h. der Füllstand in der Entgasungskolonne ist höher als in der Begasungskolonne. Über die Rückführleitung kann das Medium aus der Entgasungskolonne wieder in die Begasungskolonne rückgeführt werden.

In dem erfindungsgemäßen Verfahren ist es zudem bevorzugt, dass bei der Methanisierungsreaktion entstehendes Reaktionswasser dem Kreislauf entzogen wird.

Die Temperatur des Mediums in der Begasungskolonne liegt während dem erfindungsgemäßen Verfahren vorzugsweise in einem Bereich von 30 °C bis 70 °C.

Vorzugsweise wird in dem erfindungsgemäßen Verfahren die oben beschriebene erfindungsgemäße Vorrichtung verwendet. Alle Verfahrensschritte, die in Verbindung mit der erfindungsgemäßen Vorrichtung hierin beschrieben sind, sollen auch als mögliche Merkmale des erfindungsgemäßen Verfahrens angesehen werden, und umgekehrt.

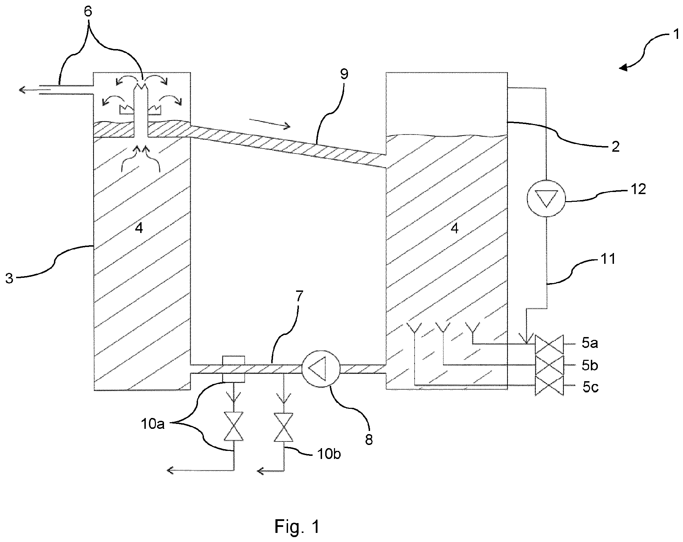

Die vorliegende Erfindung soll nun anhand der folgenden Figur 1 und des Ausführungsbeispiels erläutert werden, ist jedoch nicht auf diese spezielle Ausführungsform beschränkt.
- Figur 1: zeigt schematisch eine erfindungsgemäße Vorrichtung, die beispielsweise in dem erfindungsgemäßen Verfahren verwendet werden kann.

Figur 1 zeigt eine erfindungsgemäße Vorrichtung 1. Die Vorrichtung 1 weist auf der einen Seite eine Begasungskolonne 2 und auf der anderen Seite eine Entgasungskolonne 3 auf, die im Bereich ihrer Bodenseiten mit einer Verbindungsleitung 7 und im Bereich der oberen Seiten eine Rückführleitung 9 so verbunden sind, dass ein Medium 4 sich in einem Kreislauf zwischen der Begasungskolonne 2 und der Entgasungskolonne 3 bewegen kann. Dabei bewegt sich das Medium 4 in der Begasungskolonne 2 abwärts, wird über die Verbindungsleitung 7 in die Entgasungskolonne 3 überführt, in der es sich aufsteigend bewegt. Eine Rückführung von Medium 4 aus der Entgasungskolonne 3 in die Begasungskolonne 2 erfolgt über die Rückführleitung 9. Über eine Zuführeinrichtung 5a kann ein Wasserstoff enthaltendes Gas in das Medium 4 in der Begasungskolonne 2 eingeführt werden. Die Zuführeinrichtung 5a ist - wie in Figur 1 gezeigt - im Bereich der Bodenseite der Begasungskolonne 2 angeordnet. Das damit zugeführte Wasserstoff enthaltende Gas bewegt sich aufsteigend entgegen dem abwärts bewegenden Medium 4. Durch den höheren Druck der Flüssigkeitssäule des Mediums 4 im Bereich der Bodenseite der Begasungskolonne 2 ist das Wasserstoff enthaltende Gas in diesem Bereich besser löslich als im Bereich der oberen Seite der Begasungskolonne 2. Weiterhin kann über eine Zuführeinrichtung 5b auch ein Kohlenstoffdioxid oder Kohlenstoffmonoxid enthaltendes Gas oder eine Vorläuferverbindung davon dem Medium 4 der Begasungskolonne 2 zugeführt werden. Die Zuführung dieses Gases erfolgt auch hier vorzugsweise im Bereich der Bodenseite der Begasungskolonne 2. Weiterhin weist die Vorrichtung 1 vorzugsweise eine Zuführeinrichtung 5c für eine Mikroorganismen enthaltende Flüssigkeit auf. Die Zuführeinrichtungen 5a, 5b und 5c weisen vorzugsweise Ventile auf, über die der Zustrom der entsprechenden Gase gesteuert werden kann. Durch die Anwesenheit von methanogenen Mikroorganismen in dem Medium kann Kohlenstoffdioxid und oder Kohlenstoffmonoxid unter Bildung von Wasser in Methan umgesetzt werden. Da Methan eine bessere Löslichkeit als Wasserstoff in dem vorzugsweise wässrigen Medium 4 aufweist, wird das mit Methan angereicherte Medium 4 durch die Verbindungsleitung 7 geführt, wo es sich anschließend in der Entgasungskolonne 3 aufsteigend bewegt. Durch den abnehmenden Flüssigkeitssäulendruck in der Entgasungskolonne 3 verringert sich die Löslichkeit des Methans im Medium 4. Dadurch kann das Methan im oberen Bereich der Entgasungskolonne 3 aus dem Medium 4 ausgasen und durch eine Abführeinrichtung 6 dem Kreislauf entnommen werden. Wie in der Figur 1 gezeigt kann im Bereich der oberen Seite der Entgasungskolonne hierfür die Abführeinrichtung 6 ein Rohr mit Zackenwehr oder Zackenwehrkaskade aufweisen, durch das Medium 4 zusammen mit methanhaltigem Gas gelangen kann. Das oberhalb des Rohres des Zackenwehrs befindliche Medium 4 kann durch die vorzugsweise leicht abschüssige Rückführleitung 9 wieder in den Bereich der oberen Seite der Begasungskolonne 2 überführt werden. Das aus dem Medium 4 ausgegaste methanhaltige Gas wird dem Kreislauf über die Abführeinrichtung 6 entnommen. Eine Pumpe 8 sorgt für die Kreislaufbewegung des Mediums zwischen der Begasungskolonne 2 und der Entgasungskolonne 3. Dabei muss die Pumpe 8 nur die Energie für die Füllstanddifferenz zwischen der Entgasungskolonne 3 und der Begasungskolonne 2 aufbringen. Um entstehendes Reaktionswasser aus der Vorrichtung 1 abzuführen, kann in der Verbindungsleitung 7 eine Einrichtung 10a zum Abführen von Flüssigkeit mit Rückhalt von Mikroorganismen vorgesehen sein. Damit nur Flüssigkeit und keine anderen Mikroorganismen 4 aus dem Kreislauf entnommen wird, kann in der Einrichtung 10a beispielsweise eine Membran (nicht gezeigt) vorgesehen sein. Um Medium aus dem Kreislauf entnehmen zu können, kann auch eine Einrichtung 10b zum Abführen von Flüssigkeit (ohne Rückhalt von Mikroorganismen) vorgesehen sein. Damit das Abführen von Flüssigkeit gesteuert werden kann, können die Einrichtungen 10a und 10b Ventile aufweisen. Die Pumpe 8 kann - wie in Figur 1 gezeigt - in die Verbindungsleitung 7 integriert sein. Oberhalb der Flüssigkeitssäule des Mediums 4 in der Begasungskolonne 2 kann eine Gasentnahmeleitung 11 angebracht sein, mit der das dort befindliche Gas beispielsweise über die Zuführeinrichtung 5a in das Medium 4 zurückgeführt werden kann. Zur Steuerung dieser Rückführung muss die Gasentnahmeleitung 11 eine Gasrückführpumpe 12 aufweisen.

### Liste der Bezugszeichen:

- 1: Vorrichtung
- 2: Begasungskolonne
- 3: Entgasungskolonne
- 4: Medium
- 5a: Zuführeinrichtung für das Wasserstoff enthaltende Gas
- 5b: Zuführeinrichtung für das Kohlenstoffdioxid oder Kohlenstoffmonoxid enthaltende Gas oder einer organischen Vorläuferverbindung davon
- 5c: Zuführeinrichtung für eine Mikroorganismen enthaltende Flüssigkeit
- 6: Abführeinrichtung
- 7: Verbindungsleitung
- 8: Pumpe
- 9: Rückführleitung
- 10a: Einrichtung zum Abführen von Flüssigkeit mit Rückhalt von Mikroorganismen
- 10b: Einrichtung zum Abführen von Flüssigkeit (ohne Rückhalt von Mikroorganismen)
- 11: Gasentnahmeleitung
- 12: Gasrückführpumpe

## Patentansprüche

1. Vorrichtung (1) zur biologischen Methanisierung von Kohlenstoffmonoxid und/oder Kohlenstoffdioxid mittels methanogener Mikroorganismen durch Umsetzung von Wasserstoff und Kohlenstoffmonoxid und/oder Kohlenstoffdioxid, die Folgendes aufweist:
(a) eine Begasungskolonne (2) und eine Entgasungskolonne (3), jeweils mit einer Bodenseite und einer der Bodenseite gegenüberliegenden oberen Seite;
(b) ein in der Begasungskolonne (2) und der Entgasungskolonne (3) bereitgestelltes Medium (4) mit methanogenen Mikroorganismen;
(c) eine Zuführeinrichtung (5a) zum Zuführen eines Wasserstoff enthaltenden Gases in das Medium (4) der Begasungskolonne (2), wobei die Zuführeinrichtung (5a) im Bereich der Bodenseite der Begasungskolonne (2) angeordnet ist;
(d) eine Abführeinrichtung (6) zum Abführen eines Methan enthaltenden Gases aus der Entgasungskolonne (3);
(e) eine Verbindungsleitung (7) zwischen Begasungskolonne (2) und Entgasungskolonne (3) im Bereich der Bodenseiten;
(f) eine Pumpe (8) zum Überführen von Medium (4) über die Verbindungsleitung (7) von der Begasungskolonne (2) in die Entgasungskolonne (3); und
(g) eine Rückführleitung (9) zwischen der Begasungskolonne (2) und der Entgasungskolonne (3) im Bereich der oberen Seiten zum Rückführen von Medium (4) aus der Entgasungskolonne (3) in die Begasungskolonne (2).

2. Vorrichtung (1) nach Anspruch 1, wobei die Abführeinrichtung (6) im Bereich der oberen Seite der Entgasungskolonne (3) angeordnet ist.

3. Vorrichtung (1) nach Anspruch 1 oder 2, die eine Zuführeinrichtung (5b) zum Zuführen eines Kohlenstoffdioxid oder Kohlenstoffmonoxid enthaltenden Gases oder einer organischen Vorläuferverbindung davon, die durch im Medium (4) enthaltene Mikroorganismen in Kohlenstoffmonoxid und/oder Kohlenstoffdioxid überführt wird, in das Medium (4) der Begasungskolonne (2) aufweist, wobei die Zuführeinrichtung (5) im Bereich der Bodenseite der Begasungskolonne (2) angeordnet ist.

4. Vorrichtung (1) nach einem der Ansprüche 1 bis 3, die eine Einrichtung (10a, 10b) zum Abführen von Flüssigkeit aufweist.

5. Vorrichtung (1) nach Anspruch 4, worin die Einrichtung eine Einrichtung (10a) zum Abführen von Flüssigkeit mit Rückhalt von Mikroorganismen ist.

6. Vorrichtung (1) nach einem der Ansprüche 1 bis 5, die eine Zuführeinrichtung (5c) für eine Mikroorganismen enthaltende Flüssigkeit aufweist.

7. Vorrichtung (1) nach einem der Ansprüche 1 bis 6, die im Bereich der oberen Seite der Begasungskolonne (2) eine Gasentnahmeleitung (11) aufweist, mit der nicht umgesetzter Wasserstoff, nicht gelöstes Methan und Kohlenstoffmonoxid und/oder Kohlenstoffdioxid aus dem Bereich der oberen Seite der Begasungskolonne (2) in den Bereich der Bodenseite der Begasungskolonne (2) geführt werden.

8. Vorrichtung (1) nach einem der Ansprüche 1 bis 7, worin die Begasungskolonne (2) und/oder die Entgasungskolonne (3) flüssigkeitsdurchlässige Füllkörper aufweisen.

9. Verfahren zur biologischen Methanisierung von Kohlenstoffmonoxid und/oder Kohlenstoffdioxid in einer Vorrichtung (1) mittels methanogener Mikroorganismen als Teil eines in der Vorrichtung (1) bereitgestellten Mediums (4), **dadurch gekennzeichnet, dass** das Medium (4) in einem Kreislauf über eine Begasungskolonne (2) und eine Entgasungskolonne (3) geführt wird, wobei die Kolonnen (2, 3) jeweils über eine Verbindungsleitung (7) im Bereich ihrer Bodenseiten und über eine Rückführleitung (9) im Bereich der den Bodenseiten gegenüberliegenden oberen Seiten miteinander verbunden sind, worin das Medium (4) sich in der Begasungskolonne (2) absteigend und in der Entgasungskolonne (3) aufsteigend bewegt, worin dem Medium (4) im Bereich der Bodenseite der Begasungskolonne (2) ein Wasserstoff enthaltendes Gas zugeführt wird.

10. Verfahren nach Anspruch 9, worin als Vorrichtung (1) eine Vorrichtung (1) gemäß einem der Ansprüche 1 bis 8 verwendet wird.

## Claims

1. Apparatus (1) for the biological methanation of carbon monoxide and/or carbon dioxide by means of methanogenic microorganisms by reacting hydrogen and carbon monoxide and/or carbon dioxide, the apparatus comprising the following:
(a) a gassing column (2) and a degassing column (3), each having a bottom side and an upper side which is opposite the bottom side;
(b) a medium (4) containing methanogenic microorganisms which is provided in the gassing column (2) and the degassing column (3);
(c) a feed device (5a) for feeding a hydrogen-containing gas into the medium (4) of the gassing column (2), wherein the feed device (5a) is arranged in the region of the bottom side of the gassing column (2);
(d) a discharge device (6) for discharging a methane-containing gas from the degassing column (3);
(e) a connecting line (7) in the region of the bottom sides, between the gassing column (2) and the degassing column (3);
(f) a pump (8) for transferring the medium (4) from the gassing column (2) into the degassing column (3) via the connecting line (7); and
(g) a return line (9) in the region of the upper sides, between the gassing column (2) and the degassing column (3), for returning the medium (4) from the degassing column (3) into the gassing column (2).

2. Apparatus (1) according to claim 1, wherein the discharge device (6) is arranged in the region of the upper side of the degassing column (3).

3. Apparatus (1) according to either claim 1 or claim 2, which comprises a feed device (5b) for feeding a carbon-dioxide-containing or carbon-monoxide-containing gas or an organic precursor compound thereof into the medium (4) of the gassing column (2), which gas is converted into carbon monoxide and/or carbon dioxide by microorganisms contained in the medium (4), wherein the feed device (5) is arranged in the region of the bottom side of the gassing column (2).

4. Apparatus (1) according to any of claims 1 to 3, which comprises a device (10a, 10b) for discharging liquid.

5. Apparatus (1) according to claim 4, in which the device is a device (10a) for discharging liquid with retention of microorganisms.

6. Apparatus (1) according to any of claims 1 to 5, which comprises a feed device (5c) for a liquid containing microorganisms.

7. Apparatus (1) according to any of claims 1 to 6, which comprises a gas extraction line (11) in the region of the upper side of the gassing column (2), by means of which line unreacted hydrogen, undissolved methane and carbon monoxide and/or carbon dioxide are guided from the region of the upper side of the gassing column (2) into the region of the bottom side of the gassing column (2).

8. Apparatus (1) according to any of claims 1 to 7, in which the gassing column (2) and/or the degassing column (3) comprise liquid-permeable packing materials.

9. Process for the biological methanation of carbon monoxide and/or carbon dioxide in an apparatus (1) by means of methanogenic microorganisms as part of a medium (4) provided in the apparatus (1), **characterized in that** the medium (4) is guided in a circuit via a gassing column (2) and a degassing column (3), the columns (2, 3) being connected to one another via a connecting line (7) in the region of their bottom sides and via a return line (9) in the region of the upper sides opposite the bottom sides, in which the medium (4) moves downward in the gassing column (2) and upward in the degassing column (3), in which a hydrogen-containing gas is fed to the medium (4) in the region of the bottom side of the gassing column (2).

10. Method according to claim 9, in which the apparatus (1) used is an apparatus (1) according to any of claims 1 to 8.

## Revendications

1. Dispositif (1) pour la méthanisation biologique de monoxyde de carbone et/ou de dioxyde de carbone au moyen de micro-organismes méthanogènes par réaction d'hydrogène et de monoxyde de carbone et/ou de dioxyde de carbone, présentant ce qui suit :
(a) une colonne de gazage (2) et une colonne de dégazage (3), comportant respectivement un côté fond et un côté supérieur opposé au côté fond ;
(b) un milieu (4) fourni dans la colonne de gazage (2) et dans la colonne de dégazage (3), et comportant des micro-organismes méthanogènes ;
(c) un appareil d'alimentation (5a) pour l'alimentation d'un gaz contenant de l'hydrogène dans le milieu (4) de la colonne de gazage (2), dans lequel l'appareil d'alimentation (5a) est disposé dans la zone du côté fond de la colonne de gazage (2) ;
(d) un appareil d'évacuation (6) pour l'évacuation d'un gaz contenant du méthane de la colonne de dégazage (3) ;
(e) une conduite de raccordement (7) entre la colonne de gazage (2) et la colonne de dégazage (3) dans la zone des côtés fonds ;
(f) une pompe (8) pour la transformation du milieu (4) de la colonne de gazage (2) à la colonne de dégazage (3) par l'intermédiaire de la conduite de raccordement (7) ; et
(g) une conduite de retour (9) entre la colonne de gazage (2) et la colonne de dégazage (3) dans la zone des côtés supérieurs pour le retour du milieu (4) de la colonne de dégazage (3) à la colonne de gazage (2).

2. Dispositif (1) selon la revendication 1, dans lequel l'appareil d'évacuation (6) est disposé dans la zone du côté supérieur de la colonne de dégazage (3).

3. Dispositif (1) selon la revendication 1 ou 2, présentant un appareil d'alimentation (5b) pour l'alimentation d'un gaz contenant du dioxyde de carbone ou du monoxyde de carbone ou d'un composé précurseur organique de celui-ci, lequel est transformé en monoxyde de carbone et/ou en dioxyde de carbone par des micro-organismes contenus dans le milieu (4), dans le milieu (4) de la colonne de gazage (2), dans lequel l'appareil d'alimentation (5) est disposé dans la zone du côté fond de la colonne de gazage (2).

4. Dispositif (1) selon l'une des revendications 1 à 3, présentant un appareil (10a, 10b) pour l'évacuation de liquide.

5. Dispositif (1) selon la revendication 4, dans lequel l'appareil est un appareil (10a) pour l'évacuation de liquide avec rétention de micro-organismes.

6. Dispositif (1) selon l'une des revendications 1 à 5, présentant un appareil d'alimentation (5c) pour un liquide contenant des micro-organismes.

7. Dispositif (1) selon l'une des revendications 1 à 6, présentant dans la zone du côté supérieur de la colonne de gazage (2) une conduite de prélèvement de gaz (11) avec laquelle de l'hydrogène n'ayant pas réagi, du méthane non dissous et du monoxyde de carbone et/ou du dioxyde de carbone sont amenés de la zone du côté supérieur de la colonne de gazage (2) à la zone du côté fond de la colonne de gazage (2).

8. Dispositif (1) selon l'une des revendications 1 à 7, dans lequel la colonne de gazage (2) et/ou la colonne de dégazage (3) présentent des corps de remplissage perméables aux liquides.

9. Procédé pour la méthanisation biologique de monoxyde de carbone et/ou de dioxyde de carbone dans un dispositif (1) au moyen de micro-organismes méthanogènes sous forme de partie d'un milieu (4) fourni dans le dispositif (1), **caractérisé en ce que** le milieu (4) est amené dans un circuit par l'intermédiaire d'une colonne de gazage (2) et d'une colonne de dégazage (3), dans lequel les colonnes (2, 3) sont respectivement raccordées entre elles par l'intermédiaire d'une conduite de raccordement (7) dans la zone de leurs côtés fonds et par l'intermédiaire d'une conduite de retour (9) dans la zone des côtés supérieurs opposés aux côtés fonds, dans lequel le milieu (4) se déplace en descendant dans la colonne de gazage (2) et en montant dans la colonne de dégazage (3), dans lequel un gaz contenant de l'hydrogène est amené au milieu (4) dans la zone du côté fond de la colonne de gazage (2).

10. Procédé selon la revendication 9, dans lequel un dispositif (1) selon l'une des revendications 1 à 8 est utilisé sous forme de dispositif (1).
